(19) Europäisches Patentamt
European Patent Office
Office européen des brevets

(11) Veröffentlichungsnummer: **0 120 273 B1**

# (12) EUROPÄISCHE PATENTSCHRIFT

(45) Veröffentlichungstag der Patentschrift: **23.04.86**

(51) Int. Cl.⁴: **C 07 D 213/61**

(21) Anmeldenummer: **84101656.1**

(22) Anmeldetag: **17.02.84**

(54) **Verfahren zur kontinuierlichen Herstellung von Pentachlorpyridin.**

(30) Priorität: **26.02.83 DE 3306905**

(43) Veröffentlichungstag der Anmeldung: **03.10.84 Patentblatt 84/40**

(45) Bekanntmachung des Hinweises auf die Patenterteilung: **23.04.86 Patentblatt 86/17**

(84) Benannte Vertragsstaaten: **BE CH DE FR GB IT LI**

(56) Entgegenhaltungen:
**BE - A - 651 850**
**BE - A - 659 475**
**DE - B - 1 545 984**

**CHEMICAL ABSTRACTS, Band 96, Nr. 15, 12. April 1982, Seite 639, Nr. 122146e, Columbus, Ohio, USA; B. SHATALOV: "Heterogeneous chlorination of methane on carbon catalysts. Interrelation of the occurrence of chlorination with carbon catalyst structure";**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Bender, Herbert, Dr., Koenigsberger Strasse 5, D-6737 Boehl-Iggelheim (DE)**
Erfinder: **Eilingsfeld, Heinz, Dr., Pierstrasse 9a, D-6710 Frankenthal (DE)**
Erfinder: **Neumann, Peter, Dr., Franz-Schubert-Strasse 1, D-6908 Wiesloch (DE)**

EP 0 120 273 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Verfahren zur kontinuierlichen Herstellung von Pentachlorpyridin durch Umsetzung von Pyridin mit Chlor in der Gasphase in einem Verhältnis von höchstens 7 Mol Chlor zu 1 Mol Pyridin, mit einem Durchsatz von 3 bis 100 g Pyridin je Quadratzentimeter Katalysatorbettquerschnitt und Stunde und in Gegenwart von Koks als Katalysator mit einer spezifischen Oberfläche von höchstens 100 Quadratmeter je Gramm Koks.

In der US-PS 3583988 wird die Herstellung von Pentachlorpyridin durch Chlorierung von Piperidinen in der Gasphase in einem Strömungsrohr bei 350 bis 750° C beschrieben. Als Ausgangsstoffe kommen nur ganz oder teilweise gesättigte Heterocyclen, die bei der Chlorierung zu aromatischen, chlorierten Heterocyclen umgewandelt werden, in Frage. Man verwendet einen Überschuss an Chlor über der stöchiometrischen Menge von mindestens 30, bevorzugt 100%, bezogen auf den zu chlorierenden Stoff. Im Falle der Chlorierung von Methylpiperidin und Piperidin zu Pentachlorpyridin (Beispiele 1, 2, 7 und 8) wird die Reaktion bei einem Molverhältnis Piperidin/Chlor von 1:22-48 durchgeführt. Die erzielten Ausbeuten betragen in diesen Beispielen 30 bis 34%. Der Durchsatz errechnet sich zu 0,1 bis 1,5 $g/cm^2$ h Pyridin. Katalysatoren werden nicht verwendet. Die Ausgangsstoffe werden zusammen mit einem inerten Verdünnungsmittel, z.B. Stickstoff, durch das Reaktionsrohr geleitet.

In der DE-AS 2141632 wird die Chlorierung von ε-Caprolactam bzw. Cyclohexanonoxim bei 250 bis 600° C zu Pentachlorpyridin beschrieben. Zur Umsetzung werden in allen Beispielen 6 Mol Tetrachlorkohlenstoff, 18 bis 20 Mol Chlor pro Mol Ausgangsstoff benötigt. Die Rohausbeute betrug 24 bis 86%; ein weiterer Reinigungsschritt ist notwendig. Katalysatoren werden nicht verwendet.

Die US-PS 3420833 beschreibt die Herstellung von Pentachlorpyridin durch Chlorierung von Pyridin in Abwesenheit eines Katalysators. In einem Strömungsrohr werden Pyridin und Chlor unter turbulentem Mischen bei 400 bis 700° C zur Reaktion gebracht. Das molare Verhältnis Chlor/Pyridin liegt bevorzugt zur Herstellung des Pentachlorpyridins bei 9,3:1 bis 10,8:1 (Beispiele); als Verdünnungsmittel werden perchlorierte Kohlenwasserstoffe, in den Beispielen Tetrachlorkohlenstoff oder Tetrachlorethylen, verwendet. Der Durchsatz beträgt 0,2 bis 0,8 $g/cm^2$ h Pyridin. Man erhält in den Beispielen das Pentachlorpyridin im Gemisch mit Dichlor-, Trichlor- und Tetrachlorpyridinen.

In der DE-PS 1545984 wird eine Wirbelbettchlorierung von Pyridin in der Gasphase in einem Stickstoffstrom bei 400 bis 550° C in Gegenwart eines porösen Materials als Katalysator beschrieben. Ein molares Verhältnis von Chlor/Pyridin von mehr als 6:1, in den Beispielen 8:1 bis 9:1 wird verwendet. Als Katalysator wird in den Beispielen nur Kieselerde eingesetzt. Zwar wird Kohle als poröses Material erwähnt, aber Kieselerde oder Tonerde sind bevorzugt. Die Ausbeute an Rohprodukt beträgt 67% (Beispiel 1); nach den notwendigen Reinigungsschritten erhält man 48,6% reines Pentachlorpyrdin (Beispiel 1) bzw. 78,4% (Beispiel 4). Der Durchsatz beträgt in diesen Beispielen grössenordnungsmässig 0,1 bis 1 $g/cm^2$ h Pyridin.

In der US-PS 3370062, die eine ähnliche Umsetzung beschreibt, wird als Katalysator ein poröses Material verwendet, bei dem es sich insbesondere um Kiesel- oder Tonerde handelt. Um Ausbeuten im Bereich von 50-80% zu erzielen, sind, wie die Beispiele zeigen, 8 bis 9 Mol Chlor pro Mol Pyridin erforderlich. Lediglich in einem Beispiel (Beispiel 1) erfolgt die Umsetzung an einem Kohlekatalysator, bestehend aus Kokosnusskohle. Die spezifische Oberfläche solcher Aktivkohlekatalysatoren ist relativ gross und liegt in der Regel bei $6\text{-}12\cdot10^6$ $cm^2/g$ (s. «Ullmanns Encyklopädie d. tech. Chemie», 3. Aufl., Bd. 9, S. 807-809, 1957). Dabei werden 3,6 Mol Chlor pro Mol Pyridin bei 300-450° C und einem Durchsatz von 1,3 $g/cm^2$ h Pyridin umgesetzt. Nach Umkristallisieren werden 43 g eines Produktes erhalten, bei dem es sich den Analysendaten nach um Pentachlorpyridin handeln könnte. Diese Menge entspricht dann einer Ausbeute von nur ca. 40%.

Es wurde nun gefunden, dass man kontinuierlich Pentachlorpyridin durch Umsetzung von Pyridin mit Chlor in Gegenwart von Katalysatoren vorteilhaft herstellt, wenn man die Umsetzung in der Gasphase mit Chlor in einem Verhältnis von höchstens 7 Mol Chlor zu 1 Mol Pyridin mit einem Durchsatz von 3 bis 100 g Pyridin je Quadratzentimeter Katalysatorbettquerschnitt und Stunde und in Gegenwart von Koks als Katalysator mit einer spezifischen Oberfläche von höchstens 100 Quadratmeter je Gramm Koks durchführt.

Im Hinblick auf den Stand der Technik liefert das Verfahren nach der Erfindung auf einfacherem und wirtschaftlicherem Wege Pentachlorpyridin in besserer Ausbeute, Reinheit und Raum-Zeit-Ausbeute. Zusätzliche Lösungsmittel als Verdünnungsmittel, z.B. Halogenkohlenwasserstoffe, werden nicht benötigt. Vorteilhaft werden hohe Chlorüberschüsse und aufwendige Trenn- und Reinigungsoperationen vermieden. Da halogenärmere Nebenprodukte nicht in erheblichem Masse auftreten, ist der Betrieb einfacher und das Verfahren umweltfreundlicher. Alle diese vorteilhaften Ergebnisse sind überraschend.

Im Hinblick auf die US-PS 3583988, US-PS 3420833 und die DE-PS 1545984 ist es weiterhin überraschend, dass die erfindungsgemässen hohen Durchsätze bei niedrigen Chlorüberschüssen sehr gute Ausbeuten bei hohen Raum-Zeit-Ausbeuten ergeben würden.

Die Umsetzung kann durch die folgenden Formeln wiedergegeben werden:

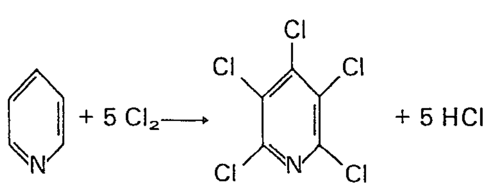

Die Ausgangsstoffe können in dem erfindungsgemässen Molverhältnis in stöchiometrischer Menge oder im Überschuss jeder Komponenten zur anderen, zweckmässig in einem Verhältnis von 5 bis 7, vorteilhaft zwischen 5 und 6, vorzugsweise von 5,05 bis 5,9, insbesondere von 5,1 bis 5,7, bevorzugt 5,2 bis 5,6, vorzugsweise 5,2 bis 5,5 Mol Chlor je Mol Pyridin verwendet werden. Die Umsetzung wird zweckmässig bei einer Temperatur von 300 bis 800, vorzugsweise 500 bis 600° C, drucklos oder unter Druck, kontinuierlich durchgeführt. Im allgemeinen werden keine zusätzlichen organischen Lösungsmittel verwendet.

Zweckmässig werden die Ausgangsstoffe in der Gasphase zusammen mit unter den Reaktionsbedingungen inerten Gasen als Verdünnungsmittel umgesetzt. Als unter den Reaktionsbedingungen inerte Gase (Inertgas) werden zweckmässig Edelgase – bevorzugt Stickstoff und/oder Kohlendioxid – und entsprechende Gemische verwendet. In einer bevorzugten Ausführungsform des Verfahrens kommen mindestens 0,1, vorzugsweise 0,5 bis 10, insbesondere von 1 bis 4 g Inertgas je Gramm Pyridin in Betracht. Bevorzugt ist eine Strömungsgeschwindigkeit des Gas/Dampf-Gemisches von Pyridin, Chlor und zweckmässig Inertgas von 0,01 bis 10, vorzugsweise 0,05 bis 0,5 m/s. Das eingesetzte Chlor kann auch im Gemisch mit Inertgasen, bevorzugt Stickstoff, verwendet werden. Desgleichen wird Pyridin zweckmässig in einem Inertgasstrom, bevorzugt Stickstoff, verdampft.

Der Durchsatz beträgt bei der Umsetzung 3 bis 100, vorteilhaft 3 bis 30 g Pyridin je Quadratzentimeter Katalysatorbettquerschnitt und Stunde.

Als Katalysator verwendet man Koks mit einer spezifischen Oberfläche von höchstens 100, zweckmässig von 0,01 bis 100, vorteilhaft 0,1 bis 80, vorzugsweise 0,2 bis 50, insbesondere 0,4 bis 10, bevorzugt 0,5 bis 2 Quadratmeter je Gramm Koks. Die spezifische Oberfläche kann mit Hilfe der BET-Methode («Ullmanns Encyklopädie der technischen Chemie», Bd. 5, S. 747-753) bestimmt werden. Man kann Koks, der bei der Verkokung anfällt, oder auch solchen Koks, der in irgendeiner Operation von chemischen Verfahren anfällt, verwenden. Unter Koks werden hier schwarze, feste und brennbare Rückstände verstanden; bezüglich der Definition, Herstellung und Eigenschaften der Kokse wird auf Roempp, «Chemielexikon» (6. Aufl.), Bd. 2, S. 3339-3342, verwiesen. So kommen Kokse, die durch Verkokung von Steinkohle, Braunkohle, Torf oder Holz hergestellt werden («Ullmanns Encyklopädie der technischen Chemie», Bd. 14, S. 491-568, 600-604), z.B. Nadelkoks, Pechkoks oder Zechenkoks, in Betracht. Auch Kokse, die aus chemischen Synthesen, z.B. der Acetylenherstellung, als Nebenstoff anfallen, können verwendet werden. Vorteilhaft sind Katalysatoren mit einer Korngrösse von 0,005 bis 3, bevorzugt 0,05 bis 1, zweckmässig 0,08 bis 1, insbesondere 0,09 bis 0,8 mm.

Bevorzugte Katalysatoren sind Zechenkoks und insbesondere Petrolkoks, zweckmässig mit einer der vorgenannten durchschnittlichen Teilchengrössen, insbesondere im Bereich von 0,08 bis 1 mm. Unter Petrolkoks werden hier solche Kohlenstoffe verstanden, die bei Krackprozessen von Erdöl, Erdölfraktionen, aliphatischen Kohlenwasserstoffen oder entsprechenden Gemischen anfallen (vgl. «Ullmanns Encyklopädie der technischen Chemie», 3. Aufl., Bd. 6, S. 625 ff.). Sie unterscheiden sich u.a. in der Art der Gewinnung, ihrer Oberfläche und katalytischen Wirksamkeit von Aktivkohlen. Die Petrolkokskatalysatoren können noch zusätzliche Aktivatoren in Gestalt von Verbindungen von Metallen der 1., 2. und/oder 8. Nebengruppe des Periodischen Systems enthalten, die z.B. durch Tränken der Katalysatoren mit Lösungen entsprechender Salze einverleibt werden. Bevorzugte Aktivatoren sind die Chloride, Sulfate, Acetate, Nitrate von Eisen, Kupfer, Zink, Platin oder Palladium. Vorteilhaft verwendet man 0,1 bis 5, insbesondere 0,3 bis 2 Gew.-% Aktivator, bezogen auf den Gesamtkatalysator und berechnet als Metall, unabhängig davon, wie er im Katalysator tatsächlich vorliegt. Vorteilhaft sind Mengen von 0,1 bis 100, bevorzugt 1 bis 10, insbesondere 1 bis 8 g Pyridin/g Katalysator und Stunde. Im allgemeinen haben die Kokskatalysatoren, insbesondere Zechenkoks und Pechkoks vorgenannter Oberflächenwerte, überraschend hohe Lebensdauer, in der Regel über 6 Monate.

Die Reaktion kann wie folgt durchgeführt werden: Man leitet kontinuierlich Chlor, Pyridindampf und gegebenenfalls Inertgas bei der Reaktionstemperatur durch den Reaktionsraum, zweckmässig ein Reaktionsrohr, der den Katalysator enthält. Dann wird aus dem Reaktionsgemisch das Pentachlorpyridin in üblicher Weise, z.B. durch Abkühlung des Gasgemisches und Filtration, abgetrennt. Vorteilhaft führt man die Abkühlung des Gasgemisches durch einen Quench mit Wasser, zweckmässig auf eine Abkühltemperatur von 10 bis 40° C, durch. Als Quench wird hier das Abkühlen der heissen Reaktionsgase mit einer Flüssigkeit (Quenchwasser) definiert. Das Quenchwasser hat vorteilhaft eine Temperatur von 5 bis 30° C. Im allgemeinen verwendet man 0,5 bis 2, vorzugsweise 0,8 bis 1,3 kg Quenchwasser je $Nm^3$ Reaktionsgas (Reaktionsgemisch und gegebenenfalls Inertgas). In einer bevorzugten Ausführungsform wird das Quenchwasser in eine Behandlungszone eindosiert, wobei Gas und die sich bildende Suspension innig vermischt werden. Die kondensierbaren bzw. in Wasser löslichen Nebenstoffe werden von dem Quenchwasser aus den heissen Reaktionsgasen abgetrennt. Die so gebildete Suspension wird dann abgesaugt und so der Endstoff abgetrennt. Überraschend wird Pentachlorpyridin unter den Bedingungen des Wasserquenchs nicht verseift und ohne weitere Reinigungsverfahren in einer Reinheit von 92 bis 99% (nach gaschromatographischer Analyse) erhalten und ist somit für die meisten Anwendungszwecke direkt verwendbar.

In einer bevorzugten Ausführungsform werden die Katalysatoren, zweckmässig in Splitt-, Gries- oder Kugelform, in der Wirbelschicht eingesetzt, wobei vorteilhaft Katalysatorteilchen mit vorgenannten Korngrössen, zweckmässig von 0,005 bis

3, insbesondere von 0,08 bis 1,0 mm, verwendet werden.

Die Schichthöhe des Katalysatorbetts (Festbett oder Wirbelbett) wird zweckmässig so gewählt, dass sich Verweilzeiten der Ausgangsstoffe II in der Kontaktschicht von 0,01 bis 50, vorteilhaft 0,01 bis 5, vorzugsweise 0,1 bis 2 Sekunden ergeben. Die genannten Gemische von Pyridin bzw. Chlor und inerten Gasen können zweckmässig bei der Durchführung des Verfahrens in einer Wirbelschicht verwendet werden, um den fluiden Zustand der Schicht zu erhalten. Man kann das Wirbelschichtverfahren aber auch ohne Inertgas mit den Ausgangsstoffen selbst als Wirbelschichtgas durchführen. Im allgemeinen kommen die vorgenannten Durchsätze an Pyridin auch für Wirbelbettverfahren in Frage. Entsprechend kann die Gesamtmenge oder eine Teilmenge der Ausgangsstoffe getrennt von dem Wirbelschichtgas in den Wirbelschichtreaktor eingeleitet werden. Man kann das Verfahren nach der Erfindung in einem einfachen oder unterteilten, offenen oder geschlossenen Wirbelschichtsystem mit und ohne Fliessstaubzirkulation durchführen. Bezüglich Reaktoren, Durchführung, Verfahrensvarianten und Reaktionsbedingungen des Wirbelschichtverfahrens wird auf «Ullmanns Encyklopädie der technischen Chemie», Bd. 1, S. 916 ff., verwiesen. Die Aufarbeitung des Reaktionsgemisches erfolgt in vorgenannter Weise. Bevorzugt erfolgt das Vermischen von Chlor und Pyridin erst im Wirbelbett.

Das nach dem Verfahren der Erfindung herstellbare Pentachlorpyridin ist ein wertvoller Ausgangsstoff für die Herstellung von Farbstoffen und Schädlingsbekämpfungsmitteln. Bezüglich der Verwendung wird auf die vorgenannten Veröffentlichungen verwiesen.

*Beispiel 1:*

300 g (3,84 Mol) Pyridin pro Stunde wurden in einem Stickstoffstrom (stündlich 600 g) in einem Verdampfer bei 170° C verdampft. Das Pyridin/Stickstoff-Gemisch wurde in einen Wirbelbettreaktor mit 60 mm lichter Weite bei 550° C eingeleitet. Kontinuierlich wurden 460 l Chlor pro Stunde direkt ins Wirbelbett mit einer Füllung aus 240 g Petrolkoks (spezifische Oberfläche 1 Quadratmeter je Gramm Koks) mit einer Körnung von 0,1 bis 0,4 mm eingeleitet. Der Durchsatz betrug 10,6 g/cm² h Pyridin. Das Molverhältnis betrug 5,4 Mol Chlor je Mol Pyridin. Aus dem Gasstrom wurde am Ende des Reaktors mit einem Wasserquench bei 25° C das Rohprodukt abgetrennt und abgesaugt. Nach 1 Stunde erhielt man 917 g (95% der Theorie) Rohprodukt, das nach gaschromatographischer Analyse Pentachlorpyridin mit einer Reinheit von 98,5% war. Die vorgenannten Ergebnisse wurden auch während 800 Stunden Betriebszeit erhalten.

*Beispiel 2:*

a) Analog Beispiel 1 wurden 395 g Pyridin mit 615 l Chlor pro Stunde bei 580° C zur Reaktion gebracht. Der Durchsatz betrug 14 g/cm² h. Das Molverhältnis betrug 5,5 Mol Chlor je Mol Pyridin. Nach der Aufarbeitung analog Beispiel 1 erhielt man 1205 g Pentachlorpyridin (96% der Theorie) vom Fp. 126° C (einmal aus Tetrachlorethylen umkristallisiert). Die vorgenannten Ergebnisse wurden auch während 750 Stunden Betriebszeit erhalten.

b) Analog Beispiel 2a) erhält man mit einem Durchsatz von 16,8 g/cm² h Pyridin dieselben Ergebnisse von Beispiel 2a).

*Beispiel 3:*

Die Umsetzung wurde analog Beispiel 2 durchgeführt, wobei anstelle von Petrolkoks 240 g Zechenkoks (spezifische Oberfläche 1 Quadratmeter je Gramm Koks) derselben Körnung verwendet wurden. Man erhielt die Ergebnisse des Beispiels 2a).

*Beispiel 4:*

Die Umsetzung wurde analog Beispiel 2 mit 200 g Acetylenkoks (Rückstand der Acetylensynthese) derselben Körnung und spezifischen Oberfläche durchgeführt. Die Ergebnisse entsprechen denen von Beispiel 2a).

## Patentanspruch

Verfahren zur kontinuierlichen Herstellung von Pentachlorpyridin durch Umsetzung von Pyridin mit Chlor in Gegenwart von Katalysatoren, dadurch gekennzeichnet, dass man die Umsetzung in der Gasphase mit Chlor in einem Verhältnis von höchstens 7 Mol Chlor zu 1 Mol Pyridin, mit einem Durchsatz von 3 bis 100 g Pyridin je Quadratzentimeter Katalysatorbettquerschnitt und Stunde und in Gegenwart von Koks als Katalysator mit einer spezifischen Oberfläche von höchstens 100 Quadratmeter je Gramm Koks durchführt.

## Claim

A process for the continuous preparation of pentachloropyridine by reacting pyridine with chlorine in the presence of a catalyst, wherein the reaction is carried out in the gas phase, the molar ratio of chlorine to pyridine being not more than 7:1, at a throughput of from 3 to 100 g of pyridine per square centimeter of catalyst bed cross-section per hour in the presence of coke having a specific surface area of not more than 100 m²/g, as catalyst.

## Revendication

Procédé de préparation en continu de pentachloropyridine par réaction sur des catalyseurs de la pyridine avec le chlore, caractérisé en ce que la réaction est effectuée en phase gazeuse, la proportion du chlore étant au maximum de 7 mol pour 1 mol de pyridine, le débit de la pyridine étant de 3 à 100 g par centimètre carré de section du lit du catalyseur et par heure et le catalyseur étant du coke avec une surface spécifique ne dépassant pas 100 m²/g.